# EUROPÄISCHE PATENTSCHRIFT

(11) **EP 0 560 114 B1**
(45) Veröffentlichungstag und Bekanntmachung des Hinweises auf die Patenterteilung: **17.04.1996**
(21) Anmeldenummer: 93102808.8
(22) Anmeldetag: 24.02.1993
(51) Int. Cl.: C07C 233/89, C07C 233/36, A61K 7/075, C11D 1/90

(54) **Wässrige flüssige Lösung eines Betains mit mindestens 40 Gew.-% Festkörpergehalt**
Betaine aqueous liquid solution with at least 40 weight-% of solid content
Solution aqueuse liquide d'une bétaine avec une teneur en solides d'au moins 40 % en poids

(30) Priorität: 09.03.1992 DE 4207386
(43) Veröffentlichungstag der Anmeldung: 15.09.1993
(73) Patentinhaber: Th. Goldschmidt AG, D-45127 Essen (DE)
(72) Erfinder: Weitemeyer, Christian, Dr., W-4300 Essen 1 (DE); Foitzik, Willi, W-4250 Bottrop (DE); Grüning, Burghard, Dr., W-4300 Essen 1 (DE); Käseborn, Hans-Dieter, W-4300 Essen 1 (DE); Begoihn, Uwe, W-4630 Bochum 6 (DE)

(56) Entgegenhaltungen:
- EP-A- 0 020 907
- EP-A- 0 243 619
- EP-A- 0 302 329
- EP-A- 0 353 580
- DE-A- 3 339 196

## Beschreibung

Die Erfindung betrifft eine wäßrige flüssige Lösung eines Betains der allgemeinen Formel 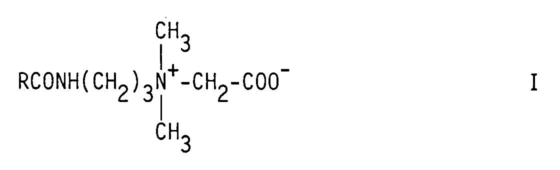
R = Alkylrest der vorzugsweise gehärteten Cocosfettsäure oder eines Fettsäuregemisches, welches im Mittel der Cocosfettsäure entspricht, wobei die Lösung einen Festkörpergehalt von mindestens 40 Gew.-%, einen pH-Wert von 5 bis 8 und einen Aminoamidgehalt von ≦ 1 Gew.-% aufweist.

Die Erfindung betrifft insbesondere wäßrige flüssige Lösungen eines Betains der vorgenannten Art mit einem Festkörpergehalt von mindestens 42 Gew.-% und besonders bevorzugt von mindestens 45 Gew.-%. Dabei ist unter Festkörpergehalt die Gewichtsmenge zu verstehen, die durch Eindampfen einer Probe auf einer flachen Glasschale während zwei Stunden bei 105°C bestimmt wird.

Es ist bekannt, daß Lösungen von Betainen der vorgenannten Art in Abhängigkeit von den zu ihrer Herstellung verwendeten Fettsäuren bzw. Fettsäuregemischen nur unterhalb einer bestimmten Konzentration an Festkörper flüssig sind. So verfestigt sich beispielsweise eine Lösung eines von der Cocosfettsäure abgeleiteten Betains bei einem Festkörvon etwa 40 Gew.-%. Aus diesem Grunde weisen handelsübliche wäßrige Lösungen des vom Cocosfett abgeleiteten Cocoamidopropylbetains Festkörperkonzentrationen auf, die deutlich unterhalb 40 Gew.-%, in den meisten Fällen bei etwa 35 Gew.-%, liegen. Mit steigender Kohlenstoffzahl sinkt die erzielbare maximale Konzentration der noch fließfähigen Lösung eines Betains. Enthält das Fettsäuregemisch mehr ungesättigte Anteile, sind häufig vergleichsweise höhere Konzentrationen erreichbar, als auf Basis gesättigter Fettsäuren.

Eine handelsübliche Betainlösung setzt sich typischerweise folgendermaßen zusammen: 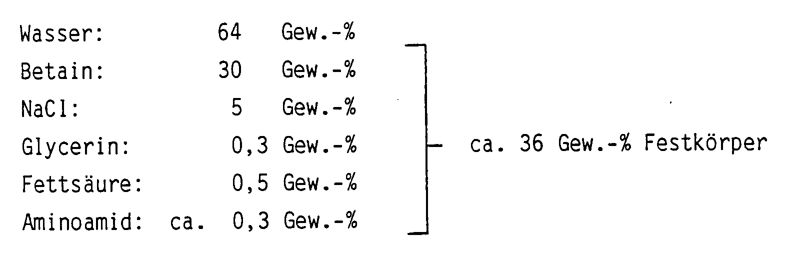
Der Festkörper stellt in diesem Sinne die Summe der Komponenten außer Wasser dar. Die Anteile des Betains und des Natriumchlorids ergeben sich aus der Stöchiometrie der Umsetzung des Fettsäureamides mit tertiären Aminogruppen (Aminoamid) und Natriumchloracetat gemäß: 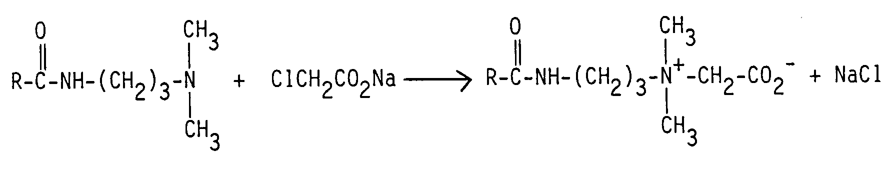
Ein kleiner Teil Aminoamid bleibt normalerweise aufgrund unvollständiger Umsetzung im Produkt, läßt sich aber durch angepaßte Stöchiometrie und Reaktionsführung auch weiter vermindern. Die weiteren aufgeführten typischen Bestandteile ergeben sich aus der Herstellung des Aminoamids:
Wenn dieses durch Umsetzung von Fettsäure und 3-N,N-Dimethylaminopropylamin gewonnen wird, sind noch restliche Anteile Fettsäure enthalten.

Wenn dieses aus Fetten und 3-N,N-Dimethylaminopropylamin hergestellt wird, sind sowohl Anteile an Fettsäure als auch an Glycerin enthalten.

Während das Aminoamid aus physiologischen Gründen eher unerwünscht ist und daher möglichst geringe Gehalte angestrebt werden, werden Glycerin und Fettsäure in kosmetischen Präparaten gern und häufig eingesetzt. An Glycerin werden in erster Linie hautpflegende Eigenschaften geschätzt. Fettsäuren weisen günstige Auswirkungen auf Formulierungen auf. So beschreibt z.B. A. L. Hunting in Cosmetics and Toiletries 97, 53 (1982) die Verwendung von Fettsäuren als Verdickungsmittel in Shampoo-Formulierungen. Sie bewirken insbesondere in Form ihrer Salze eine Viskositätserhöhung.

Es hat nicht an Versuchen gefehlt, Betainlösungen höherer Konzentration herzustellen. Dies gelang aber nur durch den Zusatz artfremder Verbindungen oder durch spezielle, das Produkt verteuernde Herstellverfahren.

So sind in der US-PS 4 243 549 gießfähige wäßrige Zubereitungen beschrieben, welche z.B. 33,5 Gew.-% eines Betains, 33,5 Gew.-% eines ethoxylierten Natriumalkylsulfates, 9 Gew.-% Kochsalz, 2 bis 3 Gew.-% Verunreinigungen, Rest Wasser enthalten. Diese Mischungen liegen in der sogenannten G-Phase vor. Die Fließfähigkeit solcher Mischungen beruht auf der speziellen mizellaren Struktur der G-Phase, die nur innerhalb eines verhältnismäßig engen Konzentrationsbereiches existent ist. Die notwendige Anwesenheit gleicher Gewichtsmengen anionischer Tenside ist jedoch bei vielen Rezepturen, in denen Betainlösungen eingesetzt werden, unerwünscht. Selbst wenn bei einer späteren Anwendung anionische Tenside zugesetzt werden, will der Anwender selbst bestimmen, welche anionischen Tenside er verwenden will. Abmischungen mit kationischen Tensiden sind wegen ihrer Wechselwirkung mit den anionischen Tensiden nicht möglich.

Die DE-PS 36 13 944 betrifft ein Verfahren zur Herstellung fließ- und pumpfähiger, mindestens 70 Gew.-% Betain enthaltender Lösungen, wobei man bei der Herstellung des Betains
a) als Salz der Halogencarbonsäure das Ammoniumsalz einsetzt,
b) die Quaternierung in einem organischen polaren Lösungsmittel, welches höchstens 20 Gew.-% Wasser enthalten darf, durchführt,
c) nach der Quaternierung das gegebenenfalls enthaltene Wasser azeotrop abdestilliert und das ausgefallene Ammoniumhalogenid abtrennt, danach
d) das Lösungsmittel ganz oder teilweise abdestilliert und
e) vor, gleichzeitig oder nach der Destillation die gewünschte Konzentration des Betains in dem anwendungstechnisch gewünschten Lösungsmittel oder Lösungsmittelgemisch einstellt.

Bei diesem Verfahren hat sich die Notwendigkeit der Verwendung von Lösungsmitteln und der Abtrennung des ausgefallenen Ammoniumchlorids z.B. durch Filtration als nachteilig erwiesen. Außerdem wurde empfohlen, der Betainlösung vorzugsweise aliphatische Diole in Mengen von 2 bis 15 Gew.-%, bezogen auf Lösung, und 0,5 bis 10 Gew.-% Ethanol zuzusetzen, um die gewünschte niedrige Viskosität zu erreichen. Auch der Gehalt an Diolen und/oder Ethanol ist nicht immer erwünscht und tolerierbar.

Gegenstand der DE-PS 37 26 322 ist ein Verfahren zur Herstellung der oben bezeichneten Betaine in Form konzentrierter wäßriger Lösungen, wobei man die nach der Quaternierung erhaltene, vorzugsweise noch heiße Lösung falls erforderlich durch Abdampfen von Wasser auf die gewünschte Konzentration einstellt und vor oder nach der Einstellung der gewünschten Konzentration der Lösung Mineralsäure in solchen Mengen zusetzt, daß der pH-Wert der Lösung 1 bis 4,5 beträgt. Hier treten aber bei Lagerung, Transport und Verwendung der sauren Betainlösungen Korrosionsprobleme auf, die die Verwendung säurefester Werkstoffe für Lagerbehälter, Tankwagen und dgl. erforderlich machen.

Schließlich sei auf die DE-PS 38 26 654 hingewiesen, wobei konzentrierte Betainlösungen dadurch erhalten werden, daß man dem Reaktionsgemisch vor oder während der Quaternierungsreaktion oder der erhaltenen Lösung des Betains nichtionogene, wasserlösliche Tenside in solchen Mengen zusetzt, daß die fertige Lösung 3 bis 20 Gew.-% nichtionogene Tenside enthält, wobei eine pH-Einstellung der Lösung durch Zugabe von Lauge auf einen pH-Wert ≧ 5 bis 9 nach der Quaternierung ausgeschlossen ist. Auch in diesem Falle enthält die Betainlösung ein artfremdes nichtionogenes Tensid, dessen Anwesenheit bei der späteren Verwendung der Betainlösungen unerwünscht sein kann.

Die vorliegende Erfindung befaßt sich mit dem technischen Problem, wäßrige flüssige Betainlösungen mit mindestens 40 Gew.-% Festkörpergehalt, vorzugsweise jedoch mindestens 42 Gew.-% und insbesondere mindestens 45 Gew.-% Festkörpergehalt und einem pH-Wert von 5 bis 8 zur Verfügung zu stellen, die frei von systemfremden Tensiden und organischen Lösungsmitteln sind und zu deren Herstellung keine zusätzlichen Verfahrensschritte, wie z.B. Filtrationen, notwendig sind.

Die Betainlösungen sollen darüber hinaus auch frei sein von Beimengungen und Verunreinigungen, die aufgrund unvollständiger Reaktion der eingesetzten Rohstoffe in den Betainlösungen verbleiben, sofern diese Restmengen aus physiologischen Gründen unerwünscht sind. Als solche Verunreinigungen sind Monochloracetat und Fettsäuredimethylaminopropylamid ("Aminoamid") zu nennen.

Die Betainlösungen sollen so konzentriert sein, daß eine besondere Konservierung der Lösungen gegen bakterielle Zersetzung nicht erforderlich ist. Dies ist ab etwa 40 Gew.-% Festkörpergehalt der Lösung gegeben.

Überraschenderweise gelingt die Lösung dieser der Erfindung zugrunde liegenden Aufgabe durch die Einstellung eines bestimmten Gehaltes an freier Fettsäure und gegebenenfalls geringer Mengen Glycerin in der Lösung des Betains.

Gegenstand der Erfindung ist somit eine wäßrige flüssige Lösung eines Betains der allgemeinen Formel 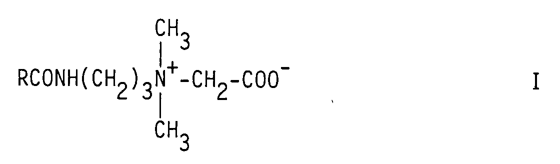
R = Alkylrest der vorzugsweise gehärteten Cocosfettsäure oder eines Fettsäuregemisches, welches im Mittel der Cocosfettsäure entspricht, wobei die Lösung einen Festkörpergehalt von mindestens 40 Gew.-%, einen pH-Wert von 5 bis 8 und einen Aminoamidgehalt von ≦ 1 Gew.-% aufweist, gekennzeichnet durch einen Gehalt an 1 bis 3 Gew.-% (bezogen auf Lösung) einer oder mehreren gesättigten Fettsäuren mit im Mittel 8 bis 18 Kohlenstoffatomen oder einer oder mehreren ungesättigten Fettsäuren mit im Mittel 8 bis 24 Kohlenstoffatomen und 0 bis 4 Gew.-% (bezogen auf Lösung) Glycerin.

Als gesättigte Fettsäuren mit im Mittel 8 bis 18 Kohlenstoffatomen kommen insbesondere die natürlich vorkommenden Fettsäuren und Fettsäuregemische in Betracht, wobei die Fettsäuren und Fettsäuregemische mit im Mittel 8 bis 12 Kohlenstoffatomen bevorzugt sind.

Als ungesättigte Fettsäuren mit im Mittel 8 bis 24 Kohlenstoffatomen sind die natürlich vorkommenden ungesättigten Fettsäuren und deren Gemische bevorzugt, wie Ölsäure, Ricinolsäure und die aus Fischöl gewonnene Fettsäure.

Besonders bevorzugt sind gehärtete und ungehärtete Cocosfettsäure, Laurinsäure, Ölsäure und Ricinolsäure.

Dabei kann die in freier Form vorliegende Fettsäure der Fettsäure RCOOH, von dem das Betain abgeleitet ist, entsprechen.

Vorzugsweise ist die erfindungsgemäße Betainlösung durch einen Gehalt an 1,5 bis 3 Gew.-% Fettsäure und durch einen Gehalt an 1 bis 2 Gew.-% Glycerin gekennzeichnet.

Unter dem Begriff Cocosfettsäure versteht der Fachmann im Handel angebotene nicht gehärtete oder gehärtete Fettsäuregemische, die aus Cocosöl gewonnen werden und im Durchschnitt folgende Zusammensetzung aufweisen:

| Cocosfettsäure: Kohlenstoffanzahl | nicht gehärtet Gew.-% | gehärtet Gew.-% |
|---|---|---|
| 6 | 0 - 1 | 0 - 1 |
| 8 | 5 - 10 | 5 - 10 |
| 10 | 3 - 10 | 5 - 10 |
| 12 | 43 - 53 | 43 - 53 |
| 14 | 15 - 22 | 15 - 22 |
| 16 | 7 - 14 | 7 - 14 |
| 18 | 2 - 8 | 4 - 12 |
| 18 1fach ungesättigt | 2 - 12 | 0 - 1 |
| 18 2fach ungesättigt | 0 - 3 | 0 |
| 18 3fach ungesättigt | 0 - 3 | 0 |

Andere Fettsäuren, wie Ricinolsäure oder Erucasäure, können in Mengen von 0 bis 3 Gew.-% enthalten sein.

Der Begriff Cocosfettsäuren umfaßt erfindungsgemäß auch die gegebenenfalls gehärteten Palmkernfettsäuren:

| Palmkernfettsäure: Kohlenstoffanzahl | nicht gehärtet Gew.-% | gehärtet Gew.-% |
|---|---|---|
| 6 | 0 - 1 | 0 - 1 |
| 8 | 3 - 6 | 3 - 6 |
| 10 | 3 - 6 | 3 - 6 |
| 12 | 40 - 52 | 40 - 52 |
| 14 | 14 - 18 | 14 - 18 |
| 16 | 6 - 14 | 6 - 14 |
| 18 | 1 - 8 | 10 - 17 |
| 18 1fach ungesättigt | 9 - 16 | 0 - 2 |
| 18 2fach ungesättigt | 1 - 3 | 0 |
| 18 3fach ungesättigt | 0 - 1 | 0 |

Bei Betainen, die von nicht gehärteten Fettsäuren hergeleitet sind, genügt im allgemeinen die Einstellung des Gehaltes an freier Fettsäure und die Einstellung des pH-Wertes, um die gewünschten konzentrierten Betainlösungen zu erhalten. Verwendet man gehärtete Cocosfettsäure oder Palmkernfettsäure, ist im allgemeinen ein Zusatz von bis zu 4 Gew.-% Glycerin zu empfehlen. Die Verwendung gehärteter Cocosfettsäure oder Palmkernfettsäure ist bevorzugt.

Beispiele erfindungsgemäßer Betainlösungen sind:

| Art des Betains R-COOH | Betain | Kochsalz | freie Fettsäure | Glycerin | Viskosität mPas, 25°C |
|---|---|---|---|---|---|
| | [ jeweils in Gew.-% ] | | | | |
| Cocosfettsäure | 36,8 | 6,2 | 1,06 | 0,89 | 85 |
| Cocosfettsäure, gehärtet | 36,0 | 6,6 | 1,55 | 0,94 | 75 |
| Palmkernfettsäure | 36,7 | 6,2 | 1,78 | 0 | 90 |

Nach einem weiteren Aspekt der vorliegenden Erfindung werden die erfindungsgemäßen Betainlösungen hergestellt durch Quaternierung einer Verbindung der allgemeinen Formel 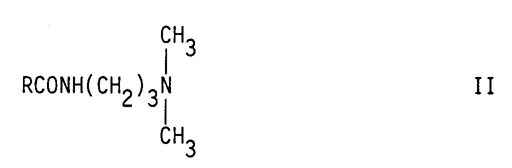
wobei R wie oben definiert ist, mit Chloressigsäure oder ihren Salzen bei erhöhten Temperaturen, wobei man für die Quaternierungsreaktion ein Fettsäureaminoamid der allgemeinen Formel II, welches die gewünschte Menge freie Fettsäure enthält, verwendet oder dem Reaktionsansatz vor oder während der Quaternierungsreaktion die gewünschte Menge an Fettsäure und dem Reaktionsansatz gegebenenfalls Glycerin zusetzt.

Unter dem Begriff der "erhöhten Reaktionstemperaturen" sind im allgemeinen Temperaturen von 80 bis 180°C zu verstehen. Wenn Temperaturen über 100°C angewendet werden, müssen die Umsetzungen in einem geschlossenen Reaktionsgefäß stattfinden. Temperaturen von 120 bis 160°C sind bevorzugt, da in diesem Temperaturbereich die Viskosität des Reaktionsmediums besonders gering ist und die Umsetzung besonders schnell abläuft.

Bei der Umsetzung wird zweckmäßig die Menge an Chloressigsäure so gewählt, daß am Ende der Reaktion das Aminoamid bis auf Reste ≦ 1 Gew.-% verbraucht ist. Es ist darüber hinaus möglich, durch Wahl eines geeigneten alkalischen pH-Wertes den Aminoamidgehalt zu verringern, wie dies in der DE-PS 29 26 479 beschrieben ist.

Vorzugsweise wird als Salz der Monochloressigsäure Natriumchloracetat verwendet, das auch im Ansatz aus Chloressigsäure und Natronlauge in situ gebildet werden kann.

Gegebenenfalls wird der pH-Wert nach Beendigung der Umsetzung vor dem Abkühlen auf Raumtemperatur mit einer geeigneten Säure auf 5 bis 8 eingestellt. Der pH-Bereich von 5 bis 7, insbesondere von 5,5 bis 6,5, ist bevorzugt.

Verwendet man bei der Herstellung der erfindungsgemäßen Betainlösungen ein Aminoamid der Formel II, welches die gewünschte Menge freie Fettsäure bereits enthält, wird diese in den meisten Fällen mit der Fettsäure RCOOH des Amidamins identisch sein. Der Gehalt an freier Fettsäure kann dann bereits bei dem Ansatz zur Herstellung des Aminoamids berücksichtigt werden. Man kann aber auch ein Aminoamid verwenden, das eine Säurezahl von 0 oder annähernd 0 aufweist und diesem eine oder mehrere gesättigte Fettsäuren mit im Mittel 8 bis 18 Kohlenstoffatomen oder eine oder mehrere ungesättigte Fettsäuren mit im Mittel 8 bis 24 Kohlenstoffatomen zusetzen.

Im Falle des Zusatzes von Glycerin kann dieser vor oder während der Quaternierungsreaktion erfolgen. Ein Zusatz nach der Quaternierungsreaktion ist zwar nicht auszuschließen, aber nicht vorteilhaft.

Die erfindungsgemäßen Betainlösungen erfüllen die Forderung, frei von fremden Tensiden zu sein und einen Aminoamidgehalt ≦ 1 Gew.-% aufzuweisen. Sie bedürfen keines besonderen Zusatzes von antimikrobiellen Wirkstoffen. Sie sind dabei in Abhängigkeit von der mittleren Kettenlänge der verwendeten Fettsäuregemische und dem Grad ihrer Ungesättigtheit bis zu einem Feststoffgehalt von etwa 48 Gew.-% flüssig.

In den folgenden Beispielen wird die Herstellung erfindungsgemäßer Betainlösungen näher erläutert.

### Beispiele

In den folgenden Beispielen werden u.a. Cocosfettsäureaminoamide, hergestellt aus verschiedenen Fettrohstoffen und 3-N,N-Dimethylaminopropylamin, sowie Natriummonochloracetat handelsüblicher Qualität verwendet.

In den Beispielen A1 bis A4 werden nichterfindungsgemäße Betainlösungen hergestellt. In den Beispielen A1 und A2 ist eine Säurezahl eingestellt worden, die jeweils außerhalb des erforderlichen Bereichs liegt, während in den ansonsten gleichartigen erfindungsgemäßen Beispielen B1 bis B3 flüssige Betainlösungen mit 45 % Festkörper erhalten werden. Im Beispiel A3 ist kein Glycerin enthalten, obwohl dies zur Erzielung eines fließfähigen Produktes notwendig gewesen wäre. Dies wird im ansonsten gleichartigen erfindungsgemäßen Beispiel B4 gezeigt. Im nichterfindungsgemäßen Beispiel A4 ist der pH-Wert nicht auf den Bereich 5 bis 8 eingestellt worden.

Die eingesetzten Fettsäureaminoamide sind gekennzeichnet durch:

| Fettsäureaminoamid A: | | |
|---|---|---|
| tertiärer Amin-Stickstoff-Gehalt: | | 4,6 % |
| Säurezahl: | | 2,1 |
| Glyceringehalt: | | 3,0 % |
| Fettsäureverteilung: | Capron- | 0,5 % |
| | Capryl- | 6,7 % |
| | Caprin- | 6,5 % |
| | Laurin- | 48,0 % |
| | Myristin- | 17,5 % |
| | Palmitin- | 12,0 % |
| | Stearin- | 8,9 % |
| | Öl- | - |
| | Linol- | - |

| Fettsäureaminoamid B: | | |
|---|---|---|
| tertiärer Amin-Stickstoff-Gehalt: | | 4,3 % |
| Säurezahl: | | 4,1 |
| Glyceringehalt: | | - |
| Fettsäureverteilung: | Capron- | 1,0 % |
| | Capryl- | 7,0 % |
| | Caprin- | 6,0 % |
| | Laurin- | 48,0 % |
| | Myristin- | 19,0 % |
| | Palmitin- | 9,0 % |
| | Stearin- | 10,0 % |
| | Öl- | - |
| | Linol- | - |

| Fettsäureaminoamid C: | | |
|---|---|---|
| tertiärer Amin-Stickstoff-Gehalt: | | 4,42 % |
| Säurezahl: | | 4,3 |
| Glyceringehalt: | | - |
| Fettsäureverteilung: | Capron- | 1,0 % |
| | Capryl- | 7,0 % |
| | Caprin- | 6,0 % |
| | Laurin- | 48,0 % |
| | Myristin- | 19,0 % |
| | Palmitin- | 9,0 % |
| | Stearin- | 2,0 % |
| | Öl- | 7,0 % |
| | Linol- | 1,0 % |

| Fettsäureaminoamid D: | | |
|---|---|---|
| tertiärer Amin-Stickstoff-Gehalt: | | 4,2 % |
| Säurezahl: | | 2,1 |
| Glyceringehalt: | | 2,9 % |
| Fettsäureverteilung: | Capron- | - |
| | Capryl- | 7,0 % |
| | Caprin- | 6,0 % |
| | Laurin- | 48,0 % |
| | Myristin- | 17,0 % |
| | Palmitin- | 7,0 % |
| | Stearin- | 3,0 % |
| | Öl- | 11,0 % |
| | Linol- | 1,0 % |

| Fettsäureaminoamid E: | | |
|---|---|---|
| tertiärer Amin-Stickstoff-Gehalt: | | 4,2 % |
| Säurezahl: | | 1,8 |
| Glyceringehalt: | | 3,3 % |
| Fettsäureverteilung: | Capron- | - |
| | Capryl- | 3,0 % |
| | Caprin- | 3,0 % |
| | Laurin- | 50,0 % |
| | Myristin- | 17,0 % |
| | Palmitin- | 12,0 % |
| | Stearin- | 5,0 % |
| | Öl- | 10,0 % |
| | Linol- | - |

| Fettsäureaminoamid F: | | |
|---|---|---|
| tertiärer Amin-Stickstoff-Gehalt: | | 4,42 % |
| Säurezahl: | | 4,3 |
| Glyceringehalt: | | 2,9 % |
| Fettsäureverteilung: | Capron- | 1,0 % |
| | Capryl- | 7,0 % |
| | Caprin- | 12,0 % |
| | Laurin- | 40,0 % |
| | Myristin- | 19,0 % |
| | Palmitin- | 12,0 % |
| | Stearin- | 9,0 % |
| | Öl- | - |
| | Linol- | - |

| Fettsäureaminoamid G: | | |
|---|---|---|
| tertiärer Amin-Stickstoff-Gehalt: | | 4,6 % |
| Säurezahl: | | 18 |
| Glyceringehalt: | | - |
| Fettsäureverteilung: | Capron- | 1,0 % |
| | Capryl- | 7,0 % |
| | Caprin- | 6,0 % |
| | Laurin- | 48,0 % |
| | Myristin- | 19,0 % |
| | Palmitin- | 9,0 % |
| | Stearin- | 2,0 % |
| | Öl- | 7,0 % |
| | Linol- | 1,0 % |

### A) Nichterfindungsgemäße Beispiele

### Beispiel A1

305 g Cocosfettsäureaminoamid A werden mit 1,1 g Cocosfettsäure einer Säurezahl (SZ) von 290 versetzt. Das entstehende Gemisch aus Cocosfettsäureaminoamid und Fettsäure weist eine SZ von 3 auf, was 0,36 % Fettsäure, berechnet als Laurinsäure, entspricht. Das Gemisch wird zu einer Lösung von 131 g 98 %igem Natriummonochloracetat (1,1 Mol) in 534 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird mit 10 %iger wäßriger Salzsäure auf pH 6 eingestellt. Es entsteht ein gelförmiges, nicht fließfähiges Produkt, das durch folgende Kennzeichen charakterisiert ist:

| | |
|---|---|
| Festkörper: | 45 % |
| Betain: | 37,2 % |
| NaCl: | 6,7 % |
| Fettsäure: | 0,1 % |
| Glycerin: | 0,97 % |
| Aminoamid: | < 0,3 % |
| pH-Wert: | 6,0 |

### Beispiel A2

305 g Cocosfettsäureaminoamid A werden mit 39 g Cocosfettsäure einer SZ von 290 versetzt. Das entstehende Gemisch aus Cocosfettsäureaminoamid und Fettsäure weist eine SZ von 35 auf, was 11,3 % Fettsäure, berechnet als Laurinsäure, entspricht. Das Gemisch wird zu einer Lösung von 131 g 98 %igem Natriummonochloracetat (1,1 Mol) in 581 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Während der Umsetzung steigt die Viskosität stark an, das Reaktionsgemisch ist nur noch schwer rührbar. Die Reaktion wird nach 8 Std. beendet. Vor dem Abkühlen wird mit 10 %iger wäßriger Salzsäure auf pH 6 eingestellt. Es entsteht ein trübes, zäh-pastöses, nicht fließfähiges Produkt, das durch folgende Kennzeichen charakterisiert ist:

| | |
|---|---|
| Festkörper: | 45 % |
| Betain: | 34,1 % |
| NaCl: | 6,2 % |
| Fettsäure: | 3,8 % |
| Glycerin: | 0,98 % |
| Aminoamid: | 0,5 % |
| pH-Wert: | 6,0 |

### Beispiel A3

326 g Cocosfettsäureaminoamid B werden mit 16,7 g Laurinsäure mit einer SZ von 290 versetzt. Das entstehende Gemisch aus Cocosfettsäureaminoamid und Fettsäure weist eine SZ von 18 auf, was 4,9 % Fettsäure, berechnet als Laurinsäure, entspricht. Das Gemisch wird zu einer Lösung von 131 g (1,1 Mol) 98 %igem Natriummonochloracetat in 579 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird mit 10 %iger wäßriger Salzsäure auf pH 6 eingestellt. Es entsteht ein gelförmiges, nicht fließfähiges Produkt, das durch folgende Kennzeichen charakterisiert ist:

| | |
|---|---|
| Festkörper: | 45 % |
| Betain: | 36,7 % |
| NaCl: | 6,2 % |
| Fettsäure: | 1,56 % |
| Glycerin: | - |
| Aminoamid: | < 0,3 % |
| pH-Wert: | 6,0 |

### Beispiel A4

305 g Cocosfettsäureaminoamid A werden mit 17,5 g Laurinsäure einer SZ von 290 versetzt. Das entstehende Gemisch aus Cocosfettsäureaminoamid und Fettsäure weist eine SZ von 18 auf, was 5,4 % Fettsäure, berechnet als Laurinsäure, entspricht. Das Gemisch wird zu einer Lösung von 131 g 98 %igem Natriummonochloracetat (1,1 Mol) in 554 g Wasser gegeben und unter Rühren auf 120°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Abschließend wird der pH-Wert nicht durch Säurezugabe verändert. Es entsteht ein klares, festes Produkt, das durch folgende Kennzeichen charakterisiert ist:

| | |
|---|---|
| Festkörper: | 45 % |
| Betain: | 35,2 % |
| NaCl: | 6,5 % |
| Fettsäure: | 1,78 % |
| Glycerin: | 1,0 % |
| Aminoamid: | < 0,3 % |
| pH-Wert: | 8,6 |

### B) Erfindungsgemäße Beispiele

### Beispiel B1

305 g Cocosfettsäureaminoamid A werden mit 11 g Cocosfettsäure einer SZ von 290 versetzt. Das entstehende Gemisch aus Cocosfettsäureaminoamid und Fettsäure weist eine SZ von 12 auf, was 3,8 % Fettsäure, berechnet als Laurinsäure, entspricht. Das Gemisch wird zu einer Lösung von 131 g 98 %igem Natriummonochloracetat (1,1 Mol) in 546 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird mit Salzsäure auf pH 5,5 eingestellt. Es entsteht ein klares, flüssiges Produkt, das durch folgende Kennzeichen charakterisiert ist:

| | |
|---|---|
| Festkörper: | 45 % |
| Betain: | 36 % |
| NaCl: | 6,6 % |
| Fettsäure: | 1,1 % |
| Glycerin: | 0,94 % |
| Aminoamid: | < 0,3 % |
| pH-Wert: | 5,5 |
| Viskosität: | 90 mPas |

### Beispiel B2

305 g Cocosfettsäureaminoamid A werden mit 17,5 g Cocosfettsäure einer SZ von 290 versetzt. Das entstehende Gemisch aus Cocosfettsäureaminoamid und Fettsäure weist eine SZ von 18 auf, was 5,7 % Fettsäure, berechnet als Laurinsäure, entspricht. Das Gemisch wird zu einer Lösung von 131 g 98 %igem Natriummonochloracetat (1,1 Mol) in 554 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird der pH-Wert mit 10 %iger wäßriger Salzsäure auf 5 eingestellt. Es entsteht ein klares, flüssiges Produkt, das durch folgende Kennzeichen charakterisiert ist:

| | |
|---|---|
| Festkörper: | 45 % |
| Betain: | 35,8 % |
| NaCl: | 6,5 % |
| Fettsäure: | 1,8 % |
| Glycerin: | 0,93 % |
| Aminoamid: | < 0,3 % |
| pH-Wert: | 5,0 |
| Viskosität: | 90 mPas |

### Beispiel B3

305 g Cocosfettsäureaminoamid A werden mit 29 g Cocosfettsäure einer SZ von 290 versetzt. Das entstehende Gemisch aus Cocosfettsäureaminoamid und Fettsäure weist eine SZ von 27 auf, was 8,7 % Fettsäure, berechnet als Laurinsäure, entspricht. Das Gemisch wird zu einer Lösung von 131 g 98 %igem Natriummonochloracetat (1,1 Mol) in 568 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird der pH-Wert mit 10 %iger wäßriger Salzsäure auf pH 6 eingestellt. Es entsteht ein klares, flüssiges Produkt, das durch folgende Kennzeichen charakterisiert ist:

| | |
|---|---|
| Festkörper: | 45 % |
| Betain: | 34,5 % |
| NaCl: | 6,3 % |
| Fettsäure: | 2,9 % |
| Glycerin: | 0,9 % |
| Aminoamid: | < 0,3 % |
| pH-Wert: | 6,0 |
| Viskosität: | 110 mPas |

### Beispiel B4

326 g Cocosfettsäureaminoamid B werden mit 17,5 g Cocosfettsäure einer SZ von 290 und 10,6 g Glycerin versetzt. Das entstehende Gemisch aus Cocosfettsäureaminoamid, Fettsäure und Glycerin weist eine SZ von 18, was 5,4 % Fettsäure, berechnet als Laurinsäure, entspricht, und einen Glyceringehalt von 3 % auf. Das Gemisch wird zu einer Lösung von 131 g 98 %igem Natriummonochloracetat (1,1 Mol) in 593 g Wasser gegeben und unter Rühren auf 98°C erhitzt. Die Reaktion wird nach 8 Std. beendet. Es entsteht ein klares, flüssiges Produkt, das durch folgende Kennzeichen charakterisiert ist:

| | |
|---|---|
| Festkörper: | 45 % |
| Betain: | 35,5 % |
| NaCl: | 6,1 % |
| Fettsäure: | 1,78 % |
| Glycerin: | 0,93 % |
| Aminoamid: | 0,7 % |
| pH-Wert: | 5,5 |
| Viskosität: | 85 mPas |

### Beispiel B5

305 g Cocosfettsäureaminoamid A werden mit 17,5 g Laurinsäure einer SZ von 290 versetzt. Das entstehende Gemisch aus Cocosfettsäureaminoamid und Fettsäure weist ein SZ von 18 auf, was 5,4 % Fettsäure, berechnet als Laurinsäure, entspricht. Das Gemisch wird zu einer Lösung von 143 g 98 %igem Natriummonochloracetat (1,2 Mol) in 569 g Wasser gegeben und unter Rühren in einem Autoklaven auf 140°C erhitzt, wobei sich ein Druck von 3,5 bar ergibt. Es ist vorteilhaft, die Reaktion bei erhöhter Temperatur durchzuführen, weil dadurch die Viskosität des Reaktionsmediums sinkt und die Reaktionszeit verkürzt wird. Die Reaktion wird nach 4 Std. beendet. Es entsteht ein klares, flüssiges Produkt, das durch folgende Kennzeichen charakterisiert ist:

| | |
|---|---|
| Festkörper: | 45 % |
| Betain: | 34,6 % |
| NaCl: | 6,9 % |
| Fettsäure: | 1,78 % |
| Glycerin: | 0,93 % |
| Aminoamid: | 0,5 % |
| pH-Wert: | 5,3 |
| Viskosität: | 90 mPas |

### Beispiel B6

317 g Cocosfettsäureaminoamid C werden mit 17,1 g ungehärteter Cocosfettsäure einer SZ von 278 versetzt. Das entstehende Gemisch aus Cocosfettsäureaminoamid und Fettsäure weist eine Säurezahl von 18 auf, was 5,2 % Fettsäure, berechnet als Laurinsäure, entspricht. Das Gemisch wird zu einer Lösung von 131 g 98 %igem Natriummonochloracetat (1,1 Mol) in 504 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird der pH-Wert mit wenigen Tropfen 10 %iger Salzsäure auf 5,5 eingestellt. Es entsteht ein klares, flüssiges Produkt, das durch folgende Kennzeichen charakterisiert ist:

| | |
|---|---|
| Festkörper: | 48 % |
| Betain: | 39 % |
| NaCl: | 6,7 % |
| Fettsäure: | 1,8 % |
| Glycerin: | - |
| Aminoamid: | 0,5 % |
| pH-Wert: | 5,5 |
| Viskosität: | 120 mPas |

### Beispiel B7

333 g Cocosfettsäureaminoamid D werden mit 11 g Laurinsäure einer SZ von 290 versetzt. Das entstehende Gemisch aus Cocosfettsäureaminoamid und Fettsäure weist eine SZ von 12 auf, was 3,2 % Fettsäure, berechnet als Laurinsäure, entspricht. Das Gemisch wird zu einer Lösung von 131 g 98 %igem Natriummonochloracetat (1,1 Mol) in 581 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird mit wenigen Tropfen 10 %iger Salzsäure auf pH 7,5 eingestellt. Es entsteht ein klares, flüssiges Produkt, das durch folgende Kennzeichen charakterisiert ist:

| | |
|---|---|
| Festkörper: | 45 % |
| Betain: | 36,8 % |
| NaCl: | 6,2 % |
| Fettsäure: | 1,06 % |
| Glycerin: | 0,89 % |
| Aminoamid: | < 0,3 % |
| pH-Wert: | 7,5 |
| Viskosität: | 75 mPas |

### Beispiel B8

326 g Cocosfettsäureaminoamid B werden mit 10,9 g Glycerin versetzt, was einem Glyceringehalt von 3,2 % entspricht. Das Gemisch wird zu einer Lösung von 131 g 98 %igem Natriummonochloracetat (1,1 Mol) in 646 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird der pH-Wert mit wenigen Tropfen 10 %iger Salzsäure auf 7 eingestellt. Es entsteht ein klares, flüssiges Produkt, das durch folgende Kennzeichen charakterisiert ist:

| | |
|---|---|
| Festkörper: | 42 % |
| Betain: | 33,3 % |
| NaCl: | 5,9 % |
| Fettsäure: | 1,8 % |
| Glycerin: | 1,0 % |
| Aminoamid: | < 0,3 % |
| pH-Wert: | 7,0 |
| Viskosität: | 75 mPas |

### Beispiel B9

317 g Fettsäureaminoamid E werden mit 24,3 g Ölsäure mit einer SZ von 200 versetzt. Das entstehende Gemisch aus Fettsäureaminoamid und Fettsäure weist eine SZ von 18 auf, was 7,1 % Fettsäure, berechnet als Laurinsäure, entspricht. Das Gemisch wird zu einer Lösung von 131 g 98 %igem Natriummonochloracetat (1,1 Mol) in 577 g Wasser gegeben und unter Rühren auf 98°C erhitzt. Die Reaktion wird nach 8 Std. beendet. Das Produkt weist einen pH-Wert von 7,5 auf. Es entsteht ein flüssiges Produkt, das durch folgende Kennzeichen charakterisiert ist:

| | |
|---|---|
| Festkörper: | 45 % |
| Betain: | 36,7 % |
| NaCl: | 6,2 % |
| Fettsäure: | 1,78 % |
| Glycerin: | - |
| Aminoamid: | 0,3 % |
| pH-Wert: | 7,5 |
| Viskosität: | 90 mPas |

### Beispiel B10

316 g Fettsäureaminoamid F werden mit 16,5 g Laurinsäure einer SZ von 290 versetzt. Das entstehende Gemisch aus Fettsäureaminoamid und Fettsäure weist eine SZ von 18 auf, was 5,4 % Fettsäure, berechnet als Laurinsäure, entspricht. Das Gemisch wird zu einer Lösung von 131 g 98 %igem Natriummonochloracetat (1,1 Mol) in 567 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird mit Salzsäure auf pH 5,5 eingestellt. Es entsteht ein klares, flüssiges Produkt, das durch folgende Kennzeichen charakterisiert ist:

| | |
|---|---|
| Festkörper: | 45 % |
| Betain: | 36,7 % |
| NaCl: | 6,3 % |
| Fettsäure: | 1,1 % |
| Glycerin: | 0,94 % |
| Aminoamid: | < 0,3 % |
| pH-Wert: | 5,5 |
| Viskosität: | 85 mPas |

### Beispiel B11

329 g Cocosfettsäureaminoamid G werden zu einer Lösung von 131 g 98 %igem Natriummonochloracetat (1,1 Mol) in 562 g Wasser gegeben und unter Rühren auf 98°C erhitzt. Durch Zugabe von wenigen Tropfen (ca. 1,5 ml) 40 %iger NaOH wird der pH-Wert während der Reaktion im Bereich von 8 bis 9 gehalten. Die Reaktion wird nach 8 Std. beendet. Vor dem Abkühlen wird der pH-Wert mit 10 %iger wäßriger Salzsäure auf 6 eingestellt. Es entsteht ein klares, flüssiges Produkt, das durch folgende Kennzeichen charakterisiert ist:

| | |
|---|---|
| Festkörper: | 45 % |
| Betain: | 36,4 % |
| NaCl: | 6,4 % |
| Fettsäure: | 1,78 % |
| Glycerin: | 0,97 % |
| Aminoamid: | 0,4 % |
| pH-Wert: | 6,0 |
| Viskosität: | 95 mPas |

### C) Konservierungsbelastungstest

### Beispiel C

Die in Beispiel B7 hergestellte Betainlösung und ihre wäßrigen Verdünnungen auf 40 % und 35 % Festkörper werden einem mikrobiologischen Belastungstest (Konservierungsbelastungstest, vgl. Wallhäußer*) unterzogen. Jeweils 10 ml dieser Proben werden mit ca. 105 Keimen folgender Mikroorganismen angeimpft:
Staphylococcus aureus
Escherichia coli
Pseudomonas aeruginosa
Candida albicans
Aspergillus niger
Candida lipolytica
Nach 1, 24 und 72 Stunden sowie nach 7 Tagen werden die Keimzahlen bestimmt. Die Ergebnisse sind in den folgenden Tabellen zusammengefaßt:
*Karl Heinz Wallhäußer:
Praxis der Sterilisation, Desinfektion, Konservierung, Keimidentifizierung, Betriebshygiene
III. Auflage, S. 336 ff., Georg Thieme Verlag, Stuttgart, 1984

### 1.) Betainlösung mit 35 % Festkörper

| Testorganismus | Keimzahl nach | | | |
|---|---|---|---|---|
| | 1 Std. | 24 Std. | 72 Std. | 7 Tagen |
| Staphylococcus aureus | 1,4.10³ | <10 | <10 | <10 |
| Escherichia coli | 1,4.10⁵ | 1,5.10⁴ | <10 | <10 |
| Pseudomonas aeruginosa | 2,6.10⁴ | 1,0.10² | 1,0.10¹ | <10 |
| Candida albicans | 1,0.10¹ | 1,0.10¹ | <10 | <10 |
| Aspergillus niger | 2,0.10⁵ | 1,0.10⁵ | 7,0.10³ | 2,0.10³ |
| Candida lipolytica | 1,1.10⁵ | 2,9.10⁴ | 3,8.10³ | 3,9.10³ |

### 2.) Betainlösung mit 40 % Festkörper

| Testorganismus | Keimzahl nach | | | |
|---|---|---|---|---|
| | 1 Std. | 24 Std. | 72 Std. | 7 Tagen |
| Staphylococcus aureus | 2,7.10³ | <10 | <10 | <10 |
| Escherichia coli | 4,9.10⁴ | 2,3.10³ | <10 | <10 |
| Pseudomonas aeruginosa | 1,3.10⁴ | 3,0.10² | <10 | <10 |
| Candida albicans | 1,0.10¹ | <10 | <10 | <10 |
| Aspergillus niger | 1,7.10⁵ | 1,2.10⁵ | 1,2.10³ | 1,0.10² |
| Candida lipolytica | 1,2.10⁵ | 2,2.10⁴ | 2,0.10³ | 5,0.10¹ |

### 3.) Betainlösung mit 45 % Festkörper

| | | | | |
|---|---|---|---|---|
| Testorganismus | Keimzahl nach | | | |

| | 1 Std. | 24 Std. | 72 Std. | 7 Tagen |
|---|---|---|---|---|
| Staphylococcus aureus | 1,0.10² | <10 | <10 | <10 |
| Escherichia coli | 4,1.10⁴ | <10 | <10 | <10 |
| Pseudomonas aeruginosa | 8,0.10³ | 4,0.10¹ | <10 | <10 |
| Candida albicans | <10 | <10 | <10 | <10 |
| Aspergillus niger | 2,3.10⁵ | 1,7.10⁵ | 2,0.10² | 2,0.10¹ |
| Candida lipolytica | 4,3.10⁴ | 2,2.10³ | <10 | <10 |

Nach Wallhäußer ist eine ausreichende Konservierung gegeben, "wenn innerhalb eines Zeitraumes von weniger als drei Wochen die eingeimpften Bakterien auf weniger als 100 Keime pro ml vermindert werden". Diese Anforderung wird am besten von der Betainlösung mit 45 % Festkörper erfüllt. Die Betainlösung mit 40 % Festkörper genügt noch den Anforderungen, während die Betainlösung mit 35 % Festkörper keine ausreichende Beständigkeit gegenüber Mikroorganismen aufweist.

## Patentansprüche

1. Wäßrige flüssige Lösung eines Betains der allgemeinen Formel 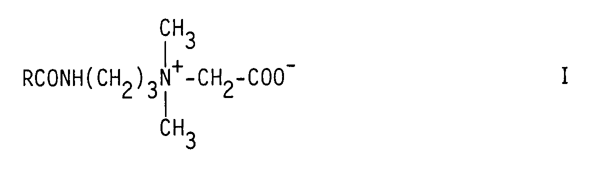 R = Alkylrest der vorzugsweise gehärteten Cocosfettsäure oder eines Fettsäuregemisches, welches im Mittel der Cocosfettsäure entspricht, wobei die Lösung einen Festkörpergehalt von mindestens 40 Gew.-%, einen pH-Wert von 5 bis 8 und einen Aminoamidgehalt von ≦ 1 Gew.-% aufweist, gekennzeichnet durch einen Gehalt an 1 bis 3 Gew.-% (bezogen auf Lösung) einer oder mehreren gesättigten Fettsäuren mit im Mittel 8 bis 18 Kohlenstoffatomen oder einer oder mehreren ungesättigten Fettsäuren mit im Mittel 8 bis 24 Kohlenstoffatomen und 0 bis 4 Gew.-% (bezogen auf Lösung) Glycerin.

2. Betainlösung nach Anspruch 1, gekennzeichnet durch einen Gehalt an 1,5 bis 3 Gew.-% Fettsäure.

3. Betainlösung nach Anspruch 1 oder 2, dadurch gekennzeichnet, daß sie als freie Fettsäure eine solche der Formel RCOOH, wobei R die bereits angegebene Bedeutung hat, enthält.

4. Betainlösung nach Anspruch 1 oder 2, dadurch gekennzeichnet, daß sie als Fettsäure Laurinsäure, Ölsäure, Ricinolsäure oder, gegebenenfalls gehärtete, Cocosfettsäure enthält.

5. Betainlösung nach einem der Ansprüche 1 bis 4, gekennzeichnet durch einen Gehalt an 1 bis 2 Gew.-% Glycerin.

6. Verfahren zur Herstellung einer Betainlösung nach einem oder mehreren der vorhergehenden Ansprüche durch Quaternierung einer Verbindung der allgemeinen Formel 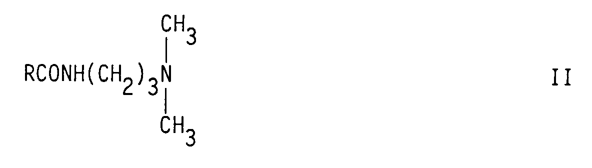 wobei R wie oben definiert ist, mit Chloressigsäure oder ihren Salzen bei erhöhten Temperaturen, dadurch gekennzeichnet, daß man für die Quaternierungsreaktion ein Fettsäureaminoamid der allgemeinen Formel II, welches die gewünschte Menge freie Fettsäure enthält, verwendet oder dem Reaktionsansatz vor oder während der Quaternierungsreaktion die gewünschte Menge an Fettsäure und dem Reaktionsansatz gegebenenfalls Glycerin zusetzt.

## Claims

1. Aqueous liquid solution of a betaine of the general formula 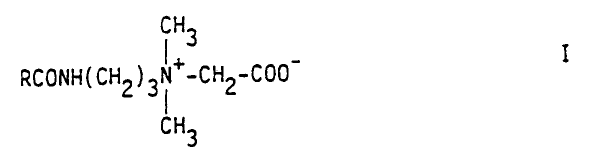 R = alkyl radical of preferably hardened coconut fatty acid or of a fatty acid mixture which on average corresponds to coconut fatty acid, the solution having a solids content of at least 40% by weight, a pH of 5 to 8 and an aminoamide content of ≦ 1% by weight, characterized in that it contains 1 to 3% by weight (based on solution) of one or more saturated fatty acids having, on average, 8 to 18 carbon atoms or one or more unsaturated fatty acids having, on average, 8 to 24 carbon atoms and 0 to 4% by weight (based on solution) of glycerol.

2. Betaine solution according to Claim 1, characterized in that it contains 1.5 to 3% by weight of fatty acid.

3. Betaine solution according to Claim 1 or 2, characterized in that, as free fatty acid, it contains one of the formula RCOOH, where R has the meaning already indicated.

4. Betaine solution according to Claim 1 or 2, characterized in that, as fatty acid, it contains lauric acid, oleic acid, ricinoleic acid or, if appropriate hardened, coconut fatty acid.

5. Betaine solution according to one of Claims 1 to 4, characterized in that it contains 1 to 2% by weight of glycerol.

6. Process for the preparation of a betaine solution according to one or more of the preceding claims by quaternization of a compound of the general formula 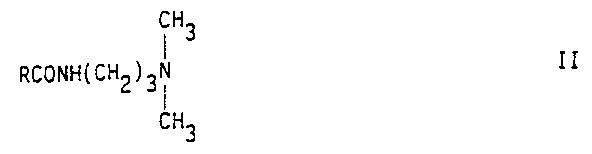 where R is as defined above, with chloroacetic acid or its salts at elevated temperatures, characterized in that a fatty acid aminoamide of the general formula II, which contains the desired amount of free fatty acid, is used for the quaternization reaction or the desired amount of fatty acid is added to the reaction mixture before or during the quaternization reaction and, if appropriate, glycerol is added to the reaction mixture.

## Revendications

1. Solution aqueuse liquide de bétaïne répondant à la formule générale 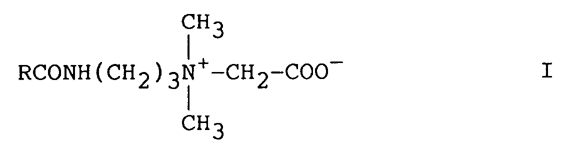 R = un reste alkyle d'acide gras de coco, de préférence durci, ou d'un mélange d'acides gras qui correspond en moyenne à l'acide gras de coco, la solution ayant une teneur en solides d'au moins 40 % en poids, une valeur de pH de 5 à 8 et une teneur en amino-amide ≦ 1 % en poids, caractérisée en ce qu'elle contient 1 à 3 % en poids (par rapport à la solution) d'un ou plusieurs acides gras saturés ayant en moyenne de 8 à 18 atomes de carbone ou d un ou plusieurs acides gras insaturés ayant en moyenne de 8 à 24 atomes de carbone et 0 à 4 % en poids de glycérol (par rapport à la solution).

2. Solution de bétaïne selon la revendication 1, caractérisée en ce qu'elle contient de 1,5 à 3 % en poids d'acide gras.

3. Solution de bétaïne selon la revendication 1 ou 2, caractérisée en ce qu'elle contient, comme acide gras libre, un acide gras répondant à la formule RCOOH où R a la signification déjà indiquée.

4. Solution de bétaïne selon la revendication 1 ou 2, caractérisée en ce qu'elle contient, comme acide gras, de l'acide laurique, de l'acide oléique, de l'acide ricinolique ou de l'acide gras de coco éventuellement durci.

5. Solution de bétaïne selon l'une des revendications 1 à 4, caractérisée en ce qu'elle contient 1 à 2 % en poids de glycérol.

6. Procédé de préparation d'une solution de bétaïne selon l'une ou plusieurs des revendications précédentes par quaternisation d'un composé répondant à la formule générale 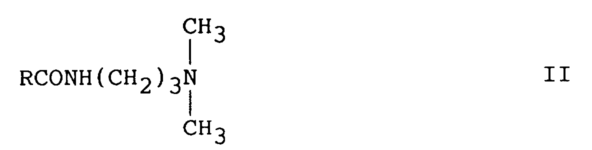 où R est tel que défini ci-dessus, avec de l'acide chloroacétique ou les sels de celui-ci, à des températures élevées, caractérisé en ce qu'on utilise, pour la réaction de quaternisation, un amino-amide d'acide gras répondant à la formule générale II, qui contient la quantité désirée d'acide gras libre, ou on ajoute la quantité désirée d'acide gras à la solution de réaction, avant ou pendant la réaction de quaternisation, et on ajoute éventuellement du glycérol à la solution de réaction.
